# EUROPÄISCHE PATENTANMELDUNG

(11) **EP 0 663 191 A2**
(43) Veröffentlichungstag der Anmeldung: **19.07.1995**
(21) Anmeldenummer: 94118040.8
(22) Anmeldetag: 15.11.1994
(51) Int. Cl.: A61C 13/00

(54) **Verfahren zur Herstellung von Formkörpern (Inserts)**

(30) Priorität: 18.11.1993 DE 4339399
(71) Anmelder: THERA Patent GmbH & Co. KG Gesellschaft für industrielle Schutzrechte, D-82229 Seefeld (DE)
(72) Erfinder: Firla, Markus, Dr., D-48147 Münster (DE); Iburg, Andreas, D-82237 Wörthsee (DE); Wanek, Erich, Dr., D-86916 Kaufering (DE)
(74) Vertreter: Müller, Bernhard, Dr. H. Schmidt & B. Müller Patentanwälte

(57) **Zusammenfassung**

Erfindungsgemäß wird ein Verfahren zur Herstellung von Formkörpern (Inserts) durch Polymerisation einer durch Licht ethylenisch polymerisierbaren Masse in Formen bereitgestellt, das dadurch gekennzeichnet ist, daß im Zuge dieser Herstellung Formkörper mit mindestens teilweise unvollständig polymerisierten Oberflächen enthalten werden. Ferner betrifft die Erfindung eine sauerstoffdurchlässige Form, die bei der Herstellung der Formkörper verwendet wird, sowie ein Kit, das die für die Durchführung des Verfahren erforderlichen Bestandteile umfaßt.

## Beschreibung

Die Erfindung betrifft vorgefertigte Formkörper (Inserts) auf der Basis ethylenisch polymerisierbarer Materialien, bevorzugt Composites, zur Befüllung der bearbeiteten Kavität eines schadhaften Zahnes.

Composite-Materialien finden aufgrund ihrer guten ästhetischen Eigenschaften in der restaurativen Zahnheilkunde vielfach Anwendung beim Befüllen von Kavitäten, vorwiegend der Klassen 3, 4 und 5. Ein Hauptproblem, das der Verwendung von Composites zum Befüllen größerer Kavitäten, vor allem im Seitenzahnbereich, entgegensteht, ist der Polymerisationsschrumpf der Materialien, der zur Bildung von Randspalten führt. Diese Randspalten zwischen dem Zahnmaterial und dem ausgehärteten Composite können verantwortlich sein für die Bildung von Sekundärkaries (US 4,744,759).

Das Ausmaß des Polymerisationsschrumpfes läßt sich um so stärker zurückdrängen, je geringer der Anteil der Composite-Harzmatrix einer Füllung und je höher der Anteil an Füllstoff in der Matrix ist. Der Füllgrad eines Composites ist aber beschränkt durch die entstehenden Handhabungsnachteile (schlechtes Anfließen, hohe Viskosität, bröselige Konsistenz). Um den Polymerisationsschrumpf zu minimieren, werden tiefe Kavitäten mit Composite sehr zeitaufwendig schichtweise gefüllt und polymerisiert.

Eine weitere Minimierung der Schrumpfungseffekte wird durch spezielle Formkörper (Inserts) erreicht, wie sie in US 4,744,759 und US 5,057,018 beschrieben werden. Diese Inserts, die aus Metall, Glas, Keramik oder Porzellan aufgebaut sein können, werden in die präparierte Zahnkavität eingebracht und mittels Composite befestigt. Ein Nachteil dieser Formkörper ist die mangelnde Verbindung, die sie mit dem Composite eingehen. Um die Haftung zu verbessern, sind zeitaufwendige Schritte wie Ätzen, Silanisieren und Bonden notwendig. Ein weiterer Nachteil besteht in den aufwendigen Herstellungsverfahren für die Formkörper.

DE 4030168 beschreibt die Verwendung lichtleitender und lichtstreuender Einsätze aus Kunststoff, Keramik, Glaskeramik, Hydroxylapatit oder dergl. Die Einsätze werden in das in eine präparierte Kavität eingefüllte Kunststoffmaterial eingebracht, das dann durch Licht gehärtet wird. Die Problematik des Verbundes von Insert und Composite wird in dieser Druckschrift nicht erörtert.

Weitere einzementierbare Formkörper aus Email, Porzellan oder Keramik sind in den Patentschriften DE 147660, DE 3620542 und DE 3743433 beschrieben. Auch bei diesen Formkörpern ist eine Folgebehandlung nach der Formgebung zur Verbesserung der Haftung notwendig (s. Prospekt PMX/MX-Ceramic-Inlay-System, Schumacher Dental Systems GmbH, Rendsburg).

Alle bisher bekannten Inserts und genormten Inlay-Körper weisen also den Nachteil auf, daß nach der Formgebung und vor der Applikation zusätzliche Verfahrensschritte, wie Ätzen, Silanisieren oder Bonden notwendig sind, um den Verbund mit dem Composite herzustellen. Die Formgebung und anschließend die Nachbehandlung der Inserts sind aufwendige Prozesse, die nicht vom Zahnarzt während der Behandlung durchführbar sind. Dies bedingt, daß in der Praxis permanent eine große Zahl von vorproduzierten Inserts in verschiedenenen Formen vorrätig sein muß, von denen der Zahnarzt nach der Präparation der Kavität das jeweils passende auswählen muß. Desweiteren verfügen diese Körper über den Nachteil, daß eine optimale Farbanpassung an die breite, bei Composites übliche Farbenpalette kaum möglich ist. Zudem unterliegt der Farbton eines Compositematerials im Laufe der Zeit infolge der im Mund herrschenden Bedingungen einer leichten Variation. Dies bedeutet, daß auch bei theoretisch optimaler Farbanpassung von Insert und Composite langsam ein Unterschied sichtbar werden kann.

Aufgabe der Erfindung war es daher, ästhetisch ansprechende Inserts zu entwickeln, die unkompliziert und zeitsparend während der Behandlung in der Praxis in der gewünschten Form und Farbe hergestellt werden, eine gute Haftung zu Composite gewährleisten und direkt nach der Formgebung appliziert werden können.

Diese Aufgaben werden gelöst, indem Inserts durch das Einbringen und Aushärten von ethylenisch polymerisierbaren Massen in Sauerstoff-permeablen Formen wie z.B. Silikonformen hergestellt werden. Die erfindungsgemäß hergestellten Formkörper weisen neben einer hohen inneren Festigkeit nicht vollständig polymerisierte Flächen auf. Diese sogenannten Schmierschichten erübrigen eine weitere Oberflächenbehandlung, da sie per se eine ausgezeichnete Haftung mit dem Composite gewährleisten.

Gegenstand der Erfindung ist ein Verfahren zur Herstellung von Formkörpern (Inserts) durch Polymerisation einer durch Licht ethylenisch polymerisierbaren Masse in Formen, das dadurch gekennzeichnet ist, daß im Zuge dieser Herstellung Formkörper mit mindestens teilweise unvollständig polymerisierten Oberflächen enthalten werden. Bevorzugt sind Inserts, die an allen Oberflächen mindestens teilweise unvollständig polymerisierte Haftschichten enthalten. Geeignet als durch Licht ethylenisch polymerisierbare Masse sind alle handelsüblichen lichthärtenden Composites für Zahnrestaurationen, wie z.B. Pertac-Hybrid, Visio-Fil oder Visio-Molar (Fa. ESPE GmbH). Geeignet sind auch dualhärtende zweikomponentige Composite, z.B. Sono-Chem (Fa. ESPE).

Die Formgebung und Polymerisation erfolgt in Formen (Matrizen) mit hoher Sauerstoff-Permeabilität, bevorzugt aus Silikon. Diese Silikonblöcke weisen an der Oberfläche ein oder mehrere Vertiefungen gleicher oder verschiedener Form und Größe auf, die als Negativformen für die Inserts dienen. Diese Vertiefungen werden z.B. dadurch erzeugt, daß vor dem Aushärten des Silikons Körper in Form der Inserts in die mit Silikon zu befüllende Gußform eingebracht und nach dem Aushärten des Silikons wieder entfernt werden. Diese Körper können aus jedem beliebigen Material bestehen, z.B. Glas, Keramik, Abform- und Dubliermasse oder ausgehärtetes Composite. Häufig können Körper mit einer geometrisch einfachen, vorgegebenen Form verwendet werden, wie sie aus dem Stand der Technik bekannt sind. Ausgehend von solchen Grundkörpern mit einfacher geometrischer Form können aber mit Hilfe der in einer Zahnarztpraxis oder in einem zahntechnischen Labor vorhandenen Werkzeuge durch mechanische Behandlung bei Bedarf auch Körper mit spezieller Form hergestellt werden. Andererseits ist es auch möglich, einen Körper durch einen Abdruck von einer bearbeiteten Kavität eines Zahnes zu erhalten. In diesem Fall läßt sich erfindungsgemäß ein Inlay mit einer Schmierschicht, die zu verbesserten Haftungseigenschaften führt, herstellen.

Es ist auch denkbar, die Kavitäten nach dem Aushärten des Silikons durch mechanische Bearbeitung wie Schneiden oder Bohren einzubringen oder eine nach dem vorstehend beschriebenen Verfahren hergestellte Form mechanisch nachzubearbeiten.

Bei Verwendung geeigneter Insert-Formen ohne Unterschnitte kann die Silikonform aufgrund ihrer Elastizität beliebig oft zur Herstellung von Inserts wiederverwendet werden, da die Form beim Herausnehmen der gehärteten Inserts nicht beschädigt wird.

In einer bevorzugten Ausführungsform der Erfindung besteht die Silikonmatrize aus einem transparenten Silikon, um eine photochemische Aushärtung und eine Kontrolle des Materials auf Blasenfreiheit zu ermöglichen. Die Form wird mit einer lichthärtenden Composite-Paste gefüllt. Die befüllten Kavitäten der Silikonmatrize werden mit lichtdurchlässiger Folie abgedeckt. Anschließend erfolgt die Aushärtung des Composites durch Belichtung. Zur Herstellung von Formkörpern mit Haftflächen auf allen Seiten wird bevorzugt zur Abdeckung eine lichtdurchlässige Silikonfolie verwendet.

Denkbar ist auch die Aushärtung durch Belichtung des Composites in Formen, die nicht durch alle Flächen sauerstoffpermeabel sind. Dies kann realisiert werden, indem z.B. Matrizen oder Deckfolien aus mindestens teilweise sauerstoffundurchlässigen Materialien verwendet werden. Dadurch entstehen ausgehärtete Inserts, deren Oberflächen höchstens teilweise unvollständig polymerisierte Schichten aufweisen.

In einer weiteren Ausführungsform der Erfindung wird ein dualhärtendes oder Paste/Paste-Composite in 2-Komponentenform angemischt, in eine sauerstoffpermeable Matrize eingebracht und mit Folie abgedeckt, die bevorzugt sauerstoffdurchlässig sein soll. Die Aushärtung kann beim dualhärtenden Composite durch Licht erfolgen. Falls die Aushärtung über den Redox-Mechanismus erfolgt, ist eine Lichtdurchlässigkeit von Matrize, bzw. Folie nicht notwendig.

Es sind auch Ausführungsformen denkbar, bei denen auf die Folie zur Abdeckung der Matrize verzichtet werden kann, jedoch erfüllt die Folie die Zwecke einer Formgebung und Glättung der Oberfläche des Inserts, eines Schutzes des Belichtungsgerätes vor Verschmutzung durch die Paste und eines Schutzes vor Verschmutzung des Inserts, beispielsweise durch Staub aus der Umgebung.

Die erfindungsgemäßen Inserts weisen den Vorteil auf, daß sie mindestens teilweise unvollständig polymerisierte Oberflächen enthalten, die als Haftflächen beim Verbund mit ethylenisch polymerisierbarem Material wirksam sind. Die Inserts können zur Befüllung von Zahnkavitäten in jedes Material einpolymerisiert werden, das den Verbund gewährleistet. Bevorzugte Materialien sind Composites, besonders bevorzugt sind Composites aus dem gleichen Material, wie die Inserts selbst. Die Haftschichten an der Oberfläche der Inserts bewirken einen festen Verbund zum Composite, ohne daß ein weiterer Behandlungsschritt wie Ätzen, Silanisieren oder Bonden nötig wäre. Die Inserts verringern beim Befüllen einer Kavität die erforderliche Menge an Composite-Paste erheblich und minimieren auf diese Art die Schrumpfung.

Ein weiterer Vorteil der erfindungsgemäßen Inserts liegt darin, daß die Formgebung und Herstellung vom Zahnarzt in kurzer Zeit ausgeführt werden kann. Der Zahnarzt kann die Inserts im Zuge einer Behandlung in der von ihm gewünschten Form herstellen und ist nicht zu einem permanenten Bereithalten von vorgefertigten Inserts verschiedener Formen gezwungen. Die erfindungsgemäßen Inserts bieten den Vorteil, daß zu ihrer Einzementierung exakt das gleiche Composite verwendet werden kann, wie zu ihrer Herstellung. Daher kann immer eine optimale Farbanpassung von Insert und Composite vorgenommen werden, so daß beide nach der Aushärtung nicht mehr unterscheidbar sind. Diese optimale ästhetische Qualität ist dauerhaft, da aufgrund der identischen Materialien nicht im Laufe der Zeit ein unterschiedliches Nachdunkeln oder eine andere ästhetische Veränderung durch Umwelteinflüsse stattfinden kann.

Ein weiterer Vorteil der erfindungsgemäßen Formkörper ist, daß nach der Einzementierung mit Composite zwischen Insert und Zement keine Unterschiede im thermischen Ausdehnungskoeffizienten aufgrund der Gleichheit der Materialien bestehen. Ein Verlust oder eine Schwächung der Haftung von Insert und Composite aufgrund unterschiedlicher thermischer Ausdehnungskoeffizienten der Materialien ist auch auf lange Zeit nicht zu befürchten.

Die ausgehärteten Inserts können auf mehrere Weisen in die vorbereitete Zahnkavität eingebracht werden. Zum einen kann zunächst der Boden der Kavität mit Composite befüllt werden. Anschließend wird das Insert eingelegt, und der Rest der Kavität mit Composite aufgefüllt. Zum anderen kann zunächst die Kavität mit Paste aufgefüllt werden. Anschließend wird das Insert eingedrückt und der Pastenüberschuß entfernt.

Nachstehend wird die Erfindung anhand von vergleichenden Beispielen näher erläutert.

### Beispiel 1: Herstellung einer transparenten Silikon-Matrize

Eine Matrize zur Herstellung von Composite-Formkörpern wurde hergestellt, indem ein transparenter Silikon-Kautschuk (KE-1300T, HEK GmbH) in einer Gußform mit den Abmessungen lxbxd von 10 cm x 4 cm x 1,5 cm nach Herstellerangaben vulkanisiert wurde. In die Gußform wurden vorher drei Formkörper aus Glas-Keramik eingebracht, die mit dem Kautschuk umgossen wurden. Nach der Aushärtung des Silikons wurden die Formkörper entfernt, wodurch an der Oberfläche des Silikons Negativformen für die Herstellung von Insert/Formkörpern mit den Abmessungen 4,1 mm x 2 mm x 1,8 mm verblieben.

Eine transparente Silikonfolie zur Abdeckung der Form wurde hergestellt, indem der gleiche Silikonkautschuk in einer Gußform mit den Abmessungen 10 cm x 4 cm x 0,9 mm vulkanisiert wurde.

### Beispiel 2: Herstellung von Composite-Inserts

Die Kavitäten der im Beispiel 1 hergestellten Silikon-Matrize wurden mit Composite-Füllungsmaterial (Pertac-Hybrid, Farbe gelb, ESPE GmbH; lichthärtendes Composite-Füllungsmaterial auf der Basis polymerisierbarer (Meth)acrylate) luftblasenfrei aufgefüllt. Anschließend wurde die Matrize mit transparenter Silikonfolie aus Beispiel 1 abgedeckt. Das Composite wurde durch einmaliges Belichten (20 sec) durch die Silikonfolie hindurch ausgehärtet, wobei der Lichtleiter mit leichtem Druck auf die Silikonfolie aufgesetzt wurde (Lampe Elipar-II, ESPE GmbH). Das entstandene Insert wurde mit einer Pinzette aus der Form entnommen, um die auf allen Oberflächen enthaltenen Haftschichten nicht zu verletzen.

### Beispiel 3: Herstellung von Probekörpern mit Composite-Inserts

Zur Simulation einer Füllung einer Zahnkavität mit Inserts und Composite und zur Bestimmung der Verbundfestigkeit beider Materialien wurden Probekörper hergestellt, indem Formen mit jeweils einem Insert aus Beispiel 2 und Composite-Paste Pertac-Hybrid Gelb (ESPE GmbH) befüllt wurden. Nach Aushärtung des Composites durch Belichtung wurde die Verbundfestigkeit von Insert und Composite überprüft, indem die Druckfestigkeit und diametrale Zugfestigkeit von mehreren Probekörpern bestimmt wurde. Die Werte sind in Tabelle 1 zusammengefaßt.

Probekörper zur Bestimmung der diametralen Zugfestigkeit wurden analog der ADA-Norm Nr. 27 hergestellt. Hierzu wurde ein Aluminiumring (3 mm Höhe, 6 mm Innendurchmesser) auf ein Deckgläschen gesetzt und mit Composite-Paste Pertac Hybrid Gelb (ESPE GmbH) aufgefüllt. Anschließend wurde jeweils ein Insert aus Beispiel 2 mit Hilfe einer Pinzette so in die Paste eingedrückt, daß es vollständig mit der Paste bedeckt war. Der Pastenüberschuß wurde mit einem Spatel entfernt. Die Form wurde unter leichtem Druck mit einem zweiten Deckgläschen oben verschlossen. Anschließend wurde je 20 Sekunden von oben und unten mit einer Lampe Elipar II bestrahlt, wobei der Lichtleiter auf das Deckglas aufgesetzt wurde. Danach wurden die Glasplatten entfernt und die Prüfkörper samt Aluminiumring 24 Stunden unter destilliertem Wasser bei 36°C aufbewahrt. Der Aluminiumring wurde entfernt und die Kanten mit Schleifpapier geglättet. Die Probekörper wurden entformt und auf der Kante stehend zwischen zwei parallele Auflageplatten einer Zwick-Universalprüfmaschine gebracht. Die diametrale Zugfestigkeit wurde mit einer Vorschubgeschwindigkeit von 1 mm/min bestimmt.

Zur Messung der Druckfestigkeit wurden zwei Halbformen aus Messing mit einem Hohlraum von 3 mm x 3 mm x 7 mm in einen Messing-Rahmen eingebracht und die Vorrichtung auf ein Deckglas gelegt. Anschließend wurde analog zur oben beschriebenen Vorgehensweise Composite und Insert eingebracht und die Vorrichtung mit einem Deckgläschen unter leichtem Anpreßdruck verschlossen.

Das Prüfgut wurde je zwanzig Sekunden mit einem Beleuchtungsgerät Elipar II auf der Oberseite und auf der gegenüberliegenden Seite ausgehärtet, wobei man den Lichtleiter direkt auf das Deckgläschen aufsetzte. Nach der manuellen Entformung wurden eventuell überstehende Prefahnen mit dem Skalpell entfernt. Die Prüfkörper wurden 24 Stunden bei 36°C im Wasserbad gelagert. Die Seitenflächen und Kanten der Prüfkörper wurden mit Schleifpapier geglättet. Die quadratischen 3x3 mm großen Deckflächen wurden mit einer Schleifmaschine planparallel geglättet. Es resultierte letztendlich ein Prüfkörper von 3 mm x 3 mm x 5 mm. Die Prüfkörper wurden zwischen die planparallelen Auflageplatten einer Zwick-Universalprüfmaschine gebracht und auf ihre Druckfestigkeit hin vermessen (Vorschubgeschwindigkeit 4mm/min).

### Vergleichsbeispiel 4

Um die Verbundfestigkeit der Probekörper aus Beispiel 3 beurteilen zu können, wurden Formen analog Beispiel 3 ausschließlich mit Composite-Paste Pertac-Hybrid Gelb gefüllt und ausgehärtet.

Die Composite-Probekörper wurden hinsichtlich Druckfestigkeit und diametraler Zugfestigkeit untersucht (Werte siehe Tabelle 1). Die Herstellung der Probekörper und Messung der diametralen Zugfestigkeit und Druckfestigkeit erfolgte analog dem in Beispiel 3 beschriebenen Verfahren. Jedoch wurden die Probekörper nur aus Composite-Paste Pertac Hybrid Gelb angefertigt und enthielten keinen Insert-Formkörper.

Ein Vergleich der Werte mit den Daten der Probekörper aus Beispiel 3 zeigt, daß die Inserts mit dem Composite über die Haftflächen einen sehr guten Verbund eingehen, da die Mittelwerte der Druck- und diametralen Zugfestigkeiten bei den Verbundprobekörpern auf vergleichbar hohem Niveau liegen, wie in reinem Composite.

Auch die Bruchcharakteristik bei der Messung der diametralen Zugfestigkeit zeigte keine Unterschiede zwischen Beispiel 3 und 4.

### Vergleichsbeispiel 5

In einem weiteren Experiment wurden schmierschichtfreie Inserts hergestellt. Hierzu wurden Inserts aus Beispiel 2 im Vakuum (2 mbar, 15 min) belichtet (Visio-β-Gerät, Fa. ESPE) und durch Bestreichen mit einer dünnen Schicht Vaseline isoliert. Anschließend wurden sie analog Beispiel 3 in Composite-paste Pertac-Hybrid eingebracht und hinsichtlich Druck- und diametraler Zugfestigkeit vermessen.

Die analog Beispiel 3 hergestellten Probekörper mit isolierten Inserts weisen wesentlich schlechtere Mittelwerte für die Druck- und diametralen Zugfestigkeiten auf (s. Tabelle 1). Dieses Beispiel zeigt, daß im Falle der Inserts aus Beispiel 3 ein Verbund mit dem Composite über die in der Silikonform entstandenen Haftschichten erfolgt.

**Tabelle 1**

| **Beispiel** | 3 | 4 | 5 |
|---|---|---|---|
| **Druckfestigkeit [MPa]** | | | |
| Mittelwert | 416 | 402 | 215 |
| Standardabweichung | 36 | | 22 |
| Varianz | 9 | | 10 |

| **diametrale Zugfestigkeit [MPa]** | | | |
|---|---|---|---|
| Mittelwert | 54 | 58 | 22 |
| Standardabweichung | 8 | 4 | 6 |
| Varianz | 15 | 6 | 27 |
| Zahl der Versuche | 10 | 10 | 7 |

## Patentansprüche

1. Verfahren zur Herstellung von Formkörpern (Inserts) durch Polymerisation einer durch Licht ethylenisch polymerisierbaren Masse in Formen, dadurch gekennzeichnet, daß im Zuge dieser Herstellung Formkörper mit mindestens teilweise unvollständig polymerisierten Oberflächen erhalten werden.

2. Verfahren nach Anspruch 1, dadurch gekennzeichnet, daß die Form aus einem sauerstoffdurchlässigen Material, insbesondere aus Silikon, besteht.

3. Verfahren nach einem der Ansprüche 1 oder 2, dadurch gekennzeichnet, daß die Form mehrteilig ist, wobei vorzugsweise ein Teil der Form aus einer Folie zur Abdeckung besteht.

4. Verfahren nach den Ansprüchen 1 bis 3, dadurch gekennzeichnet, daß mindestens ein Teil der Form lichtdurchlässig ist.

5. Verfahren zur Herstellung von Formkörpern nach einem der Ansprüche 1 bis 4, dadurch gekennzeichnet, daß es folgende Stufen umfaßt:
(a) die in einer Silikon-Matrize ausgebildeten Negativformen werden mit einer mit Hilfe von Licht ethylenisch polymerisierbaren Masse aufgefüllt und gegebenenfalls abgedeckt;
(b) die polymerisierbare Masse wird polymerisiert;
(c) die Formkörper werden aus der Silikon-Matrize entnommen.

6. Verfahren nach Anspruch 5, dadurch gekennzeichnet, daß die Silikon-Matrize hergestellt wird, indem
(a) Körper in Form der Inserts in eine mit Silikon zu befüllende Gußform eingebracht werden;
(b) die Gußform mit Silikon gefüllt und das Silikon ausgehärtet wird;
(c) die Silikon-Matrize aus der Gußform entnommen wird und die umgegossenen Körper entfernt werden.

7. Verfahren nach Anspruch 6, dadurch gekennzeichnet, daß der in Stufe (a) verwendete Körper durch einen Abdruck der bearbeiteten Kavität eines Zahnes erhalten wird.

8. Verfahren zur Herstellung von Formkörpern nach Anspruch 5, dadurch gekennzeichnet, daß die Silikon-Matrize hergestellt wird, indem in einen Silikonblock Kavitäten in Form der Inserts eingebracht werden.

9. Verfahren nach Anspruch 6, dadurch gekennzeichnet, daß es zusätzlich eine Stufe zur Herstellung einer Abdeckung der Silikon-Matrize umfaßt, bei der Silikonkautschuk in einer Gußform vulkanisiert wird.

10. Verfahren nach einem der Ansprüche 1 bis 9, dadurch gekennzeichnet, daß es sich bei der lichthärtenden ethylenisch ungesättigten Masse um ein dentales Composite handelt.

11. Kit für die Herstellung von Formkörpern nach einem der Ansprüche 1 bis 10, das folgende Bestandteile umfaßt:
(a) eine Silikongießmasse;
(b) einen als Gußform für die Silikonmasse dienenden Behälter;
(c) gegebenenfalls eine vorgefertigte Abdeckfolie für die Form;
(d) Grundkörper, die direkt oder in bearbeiteter Form als Muster für die Formkörper dienen; und
(e) ein Composite zur Herstellung der Formkörper.

12. Form zur Herstellung von Formkörpern nach einem Verfahren nach den Ansprüchen 1 bis 10.
